# EUROPEAN PATENT SPECIFICATION

(11) **EP 2 210 602 B1**
(45) Date of publication and mention of the grant of the patent: **20.05.2015**
(21) Application number: 10151046.9
(22) Date of filing: 19.01.2010
(51) Int. Cl.: A61K 31/198, A61K 38/48, A61P 11/00, A61P 11/10, A61P 11/12, A61P 11/14, A61K 45/06

(54) **Compositions for the treatment of symptoms associated with respiratory inflammations**
Zusammensetzungen zur Behandlung von Symptomen im Zusammenhang mit Atemwegsentzündungen
Compositions pour le traitement des symptômes associés aux inflammations respiratoires

(30) Priority: 22.01.2009 IT MI20090066
(43) Date of publication of application: 28.07.2010
(73) Proprietor: Wellness Industries S.r.l., 20123 Milano (IT)
(72) Inventor: Pezzini, Pietro, 46100, MANTOVA (IT)
(74) Representative: Minoja, Fabrizio

(56) References cited:
- ANONYMOUS: "Solaray Total Cleanse Respiratory" , 8 April 2008 (2008-04-08), XP002556356 Retrieved from the Internet: URL:http://web.archive.org/web/20080408113 036/http://www.smartbomb.com/slr31475.html >
- MAJIMA Y: "Mucoactive medications and airway disease" PAEDIATRIC RESPIRATORY REVIEWS 2002 GB, vol. 3, no. 2, 2002, pages 104-109, XP002556358 ISSN: 1526-0550
- DATABASE WPI Week 199710 Thomson Scientific, London, GB; AN 1997-103654 XP002556447 & JP 08 337532 A (TAKEDA CHEM IND LTD) 24 December 1996 (1996-12-24)
- DATABASE WPI Week 200167 Thomson Scientific, London, GB; AN 2001-592608 XP002556448 & JP 2001 181206 A (TAISHO PHARM CO LTD) 3 July 2001 (2001-07-03)
- RENOVANZ H -D: "Drugs with action on the bronchial tree" MEDIZINISCHE MONATSSCHRIFT FUR PHARMAZEUTEN 1984 DE, vol. 7, no. 8, 1984, pages 240-243, XP008115148 ISSN: 0342-9601
- POLU J M ET AL: "Mucolytic agents and associated therapies" REVUE DU PRATICIEN - MEDECINE GENERALE 1991 FR, vol. 5, no. 160, 1991, pages 2963-2965, XP008115149 ISSN: 0989-2737
- BARBERA S ET AL: "Multicentre clinical study on seaprose S in acute and chronic respiratory inflammation" MINERVA PNEUMOLOGICA 199612 IT, vol. 35, no. 4, December 1996 (1996-12), pages 149-156, XP008121030 ISSN: 0026-4954
- MORETTI M ET AL: "Effects of seaprose on sputum biochemical components in chronic bronchitic patients: A double-blind study vs. placebo" INTERNATIONAL JOURNAL OF CLINICAL PHARMACOLOGY RESEARCH, vol. 13, no. 5, 1993, pages 275-280, XP008121032 ISSN: 0251-1649
- Rana N: "New frontiers in Enzyme Supplementation" internet 16 February 2006 (2006-02-16), XP002576387 Retrieved from the Internet: URL:http://web.archive.org/web/20071228234 305/http://vitanetonline.com/forums/1/Thre ad/860> [retrieved on 2010-03-30]
- ANONYMOUS: "Bioinflammatory Plus" , 1 November 2006 (2006-11-01), XP002556357 Retrieved from the Internet: URL:http://web.archive.org/web/20061101025 344/http://centerofhealth.com/store/Biogen esis_Bio_Inflamm_Plus.html>
- ROGERS DUNCAN F: "Mucoactive agents for airway mucus hypersecretory diseases.", RESPIRATORY CARE SEP 2007, vol. 52, no. 9, September 2007 (2007-09), pages 1176-1193 ; di, ISSN: 0020-1324
- JÄNNE J ET AL: "Polyamines in rapid growth and cancer.", BIOCHIMICA ET BIOPHYSICA ACTA 6 APR 1978, vol. 473, no. 3-4, 6 April 1978 (1978-04-06), pages 241-293, ISSN: 0006-3002

## Description

The present invention relates to the use of N-acetyl-L-cysteine combined with promelase, a concentrated peptidase, for the preparation of compositions useful in the treatment of symptoms typical of inflammations of the upper and lower airways, including inflammations of infectious or allergic origin.

### PRIOR ART

It is known that N-acetyl-L-cysteine (NAC, (R)-2-acetamido-3-mercaptopropanoic acid), the acetylated variant of the aminoacid L-cysteine, can interact with free oxygen radicals and cross the cell membranes easily due to the presence of a sulphur-hydrogen free (-SH) thiol group. In the cells, N-acetylcysteine is deacetylated, releasing L-cysteine, an aminoacid essential for endocellular synthesis of glutathione (GSH), a highly reactive tripeptide consisting of cysteine, glycine and glutamic acid. Of said components, cysteine is the least common in foodstuffs, and is rapidly broken down in the body; its exogenous administration in the form of N-acetyl-L-cysteine or other derivatives can therefore be crucial to ensure that adequate tissue levels of glutathione are maintained. GSH is widespread in animal tissues and organs, where it plays an essential role in maintaining the cell morphology and functionality, because it constitutes the most important intracellular defence mechanism against exogenous and endogenous oxidising agents and towards numerous cytotoxic substances.

In view of the great importance of oxidative stress in numerous diseases, reduced glutathione is essential to remove free radicals from the body. In addition to its antioxidant action, glutathione possesses a strong decontaminating activity essential to protect the organs and apparatuses against potentially toxic agents: this action is particularly significant in the airways, which are exposed to continuous contact with dust particles, smog and other harmful substances inhaled from the exterior. N-acetyl-L-cysteine (NAC) also performs an intense mucolytic/fluidifying action on mucous and mucopurulent secretions, and by breaking the disulphide bridges, depolymerises the mucoprotein complexes and nucleic acids that give viscosity to the vitreous and purulent component of the sputum and other secretions. N-acetyl-L-cysteine and other cysteine derivatives (such as carboxymethyl cysteine) are therefore widely used as mucolytics/fluidifiers in disorders of the upper and lower airways. However, some important new therapeutic prospects have been opened up for the use of N-acetylcysteine in chronic infectious diseases of the airways supported by biofilm-forming bacteria, which are almost impossible to eradicate with ordinary antibiotics. Biofilm formation is one of the main methods of resistance mobilised against antibiotics by some bacteria. Biofilms are complex, organised structures, consisting of microcolonies surrounded by a polysaccharide matrix in which tiny channels of water are anastomosed to form a kind of primitive circulatory system. Biofilms can organise on the surface of different mucous membranes or on the surface of medical devices implanted or inserted in the body.

Signalling molecules produced by micro-organisms influence the formation and development of biofilms and the interaction between the micro-organisms. Due to the presence of the polysaccharide casing, which acts as a protective system against drug penetration, the micro-organisms present in the biofilm exhibit increased resistance to the host's immune defences and to antibiotic treatment. Consequently, biofilms are very important to the health, in view of their role in many chronic infections and their importance in a large number of infections caused by biomedical implants; these recurrent infections seem to be untreatable with ordinary antibiotics. Many attempts have therefore been made to develop molecules and protocols able to inhibit the growth of biofilm-producing bacteria. NAC has demonstrated good activity in inhibiting bacterial adherence and dissolving the biofilm matrix, and is therefore an important option which can be associated with chemotherapy drugs in the treatment of antibiotic-resistant infections. This possibility was confirmed by recent clinical trials, which demonstrated the efficacy of acetylcysteine in disintegrating and reducing the number of viable forms of bacteria present in biofilms of *Staphylococcus aureus* and *Escherichia coli.* These studies demonstrated the efficacy of NAC in synergising with two well-known antibiotics, thiamfenicol and fosfomycin trometamol, used in respiratory infections and uncomplicated urinary infections.

Equally well-known are the pharmacological activities of some proteases, such as serratiopeptidase, promelase (trade name Seaprose S) and bromelain, in the treatment of local inflammation, pain and oedema in numerous clinical syndromes. These enzymes, produced industrially by fermentation of bacteria (*Serratia*) or fungi (*Aspergillaceae*), or by extraction from plant sources (pineapple stalk, fermented papaya, etc.), perform an intense proteolytic, fibrinolytic and anti-bradykinin action. Bradykinin is a peptide hormone produced locally in the body tissues as a reaction to trauma; it increases the permeability of the blood vessels, dilates them, and causes the smooth muscle cells to contract. Said hormone also plays an important part in pain transmission. An excessive concentration of bradykinin is responsible for the typical symptoms of inflammation, such as swelling, reddening, hyperthermia and pain. By acting on bradykinin, proteases therefore perform an intense anti-oedema and anti-inflammatory action. The potent proteolytic and fibrinolytic activity of the peptidases also causes lysis of altered fibrinous exudates and protein substrates, thus facilitating the resorption of traumatic haematomas, improving the circulation at the focus of inflammation, and facilitating the elimination of liquefied material and the penetration of antibiotics. There is consequently a synergy between proteases and antibiotics, partly due to more specific activities recently studied in relation to the increasingly common problem of bacterial resistance.

As stated in relation to N-acetyl-L-cysteine, biofilms are one of the main means whereby bacteria defend themselves against antibiotics, and the growing phenomenon of bacterial resistance is one of the major problems faced by the international medical community, because the possibilities of developing new and more powerful anti-infective drugs seem to have been exhausted. In this context, some authors have presented data in support of the hypothesis that proteolytic enzymes, by attacking the protein matrix of biofilms, may increase the efficacy of antibiotics. This initial finding was subsequently confirmed in studies conducted *in vivo* and in clinical trials, which demonstrated that peptidases effectively eradicate infections caused by biofilm-forming bacteria and significantly increase the tissue penetration and activity of antibiotics, with the result that they are useful in the treatment of antibiotic-resistant infections.

Finally, proteases fluidify the thick, viscous secretions of the respiratory tract and, due to their characteristic proteolytic action, break down the mucoproteins that constitute the colloidal component of the bronchial and nasal mucus into simple peptides or single aminoacids. Proteases have been widely used for many years, in Europe and elsewhere, as enzymatic antiinflammatories, for the treatment of clinical syndromes characterised by local inflammation of the airways, including
- acute and chronic sinusitis
- acute and chronic bronchitis
- bronchial hypersecretion
- pharyngotonsillitis
- tracheitis
- laryngitis
- otitis
sometimes combined with antibiotic treatments.

With time, proteolytic enzymes have become accepted as anti-inflammatory agents, partly due to the absence of stomach-damaging and toxic side effects, which represent a highly problematic aspect of treatments based on non-steroidal anti-inflammatory drugs and corticosteroids. Proteases guarantee a better safety, tolerability and pharmacological manageability profile than said compounds.

### DESCRIPTION OF THE INVENTION

It has now been found that N-acetyl-L-cysteine and promelase, a proteolytic enzyme with peptidase activity, interact synergically to produce beneficial effects against a wide range of symptoms typical of inflammations and infections of the upper and lower airways: abnormal viscoelasticity of the mucous secretions, reduced mucociliary clearance, coughing, inflammation, local oedema and pain, and alteration of epithelial trophism.

The compositions according to the invention are useful as diet and nutritional supplements, as medicaments and/or as medical devices for the treatment of disorders associated with respiratory inflammations or infections, such as cell oxidation phenomena, hypersecretion and excessive viscosity of the mucus, stagnation thereof in various parts of the body, local inflammation, and the onset of infections resistant to the most common antibiotics.

The present invention therefore relates to the use of:
a) N-acetyl-L-cysteine and
b) the proteolytic enzyme with peptidase activity, promelase,
for the preparation of compositions for the treatment and prevention of symptoms associated with acute and chronic inflammations and infections of the upper and lower airways.

Also disclosed are all the proteases generally obtainable by bacterial or plant fermentation processes, such as those derived from various genera of *Aspergillus*, especially the semi-alkaline proteases isolated from the micro-organisms *Serratia marcescens, Serratia E15* and *Aspergillus melleus* (e.g.: Serrazimes®). Examples of said peptidases include promelase, serratiopeptidase and Serrazimes®.

The use of Promelase and Serrazimes® is preferred; they are readily available on the market and have long been used as anti-inflammatory and anti-oedema drugs.

The compositions according to the invention will contain the two active components in unit doses that fall into the following dose ranges:
a) N-acetyl-L-cysteine: from approx. 50 to approx. 1.500 mg, and
b) promelase from approx. 1 mg to approx. 1 g,

According to a particularly preferred aspect, the compositions according to the invention will contain the two active components in the following dose ranges:
a) N-acetyl-L-cysteine: 200-600 mg, and
b) promelase 10-100 mg.

If desired, the compositions may contain other active ingredients, with a complementary action or in any event an action useful for the purposes of the invention, such as extracts of Echinacea or other plants and/or substances whose activity in respiratory disorders is known; anti-inflammatory or mucolytic agents; minerals, vitamins, aminoacids; bacterial lysates; glucans.

The compositions according to the invention can be administered orally in a variety of forms, such as tablets, capsules, effervescent granulates, suspensions, solutions, syrups, controlled-release forms and the like; or by inhalation in the form of solutions, gel or powders.

The compositions according to the invention, administered one to three times a day, produce surprisingly favourable effects on the fluidification of the mucous secretions of the respiratory tract, mucociliary transport, local inflammation and oedema, with a significantly and unexpectedly greater effect than that obtainable by administering the individual ingredients separately, as demonstrated by the evaluation of subjective symptoms before and after treatment of volunteers with acute and chronic inflammations of the airways.

The compositions according to the invention are therefore indicated for the treatment and prevention of symptoms associated with acute and chronic inflammations and infections of the upper and lower airways, such as altered trophism and oxidation of the mucous membranes; hypersecretion of the bronchial and nasal mucus, its abnormal viscoelasticity and reduced transport; coughing; local inflammation, oedema and pain; and the presence of antibiotic-resistant infections.

The compositions will be prepared according to conventional methods well known in pharmaceutical technology, such as those described in "Remington's Pharmaceutical Handbook", Mack Publishing Co., N.Y., USA, together with commonly used excipients.

Some examples of formulations which illustrate the preparations more clearly are set out below:

### Example 1. 3 g sachets - Daily dose: 1-3 sachets

| **Name of component** | **mg / sachet** | **%** |
|---|---|---|
| Fructose | 2.393.500 | 79.783 |
| N-acetyl-L-cysteine | 200.000 | 6.667 |
| Citric acid | 150.000 | 5.000 |
| Pineapple flavouring | 120.000 | 4.000 |
| Serrazimes® | 85.000 | 2.833 |
| Silicon dioxide | 30.000 | 1.000 |
| Acesulfame K | 20.000 | 0.667 |
| E102 colouring | 1.500 | 0.050 |

### Example 2. 3 g sachets - Daily dose: 1-3 sachets

| **Name of component** | **mg / sachet** | **%** |
|---|---|---|
| Fructose | 2.401.000 | 80.033 |
| N-acetyl-L-cysteine | 200.000 | 6.667 |
| Citric acid | 150.000 | 5.000 |
| Pineapple flavouring | 120.000 | 4.000 |
| Concentrated peptidase (Serrazimes®) | 33.000 | 1.100 |
| Silicon dioxide | 30.000 | 1.000 |
| Acesulfame K | 20.000 | 0.667 |
| Microcrystalline cellulose | 13.200 | 0.440 |
| Safflower | 8.000 | 0.267 |

### Example 3. 3 g sachets - Daily dose: 1-3 sachets (Reference example)

| **Name of component** | **mg / sachet** | **%** |
|---|---|---|
| Fructose | 2.401.000 | 80.033 |
| N-acetyl-L-cysteine | 200.000 | 6.667 |
| Citric acid | 150.000 | 5.000 |
| Pineapple flavouring | 120.000 | 4.000 |
| Serratiopeptidase | 33.000 | 1.100 |
| Silicon dioxide | 30.000 | 1.000 |
| Acesulfame K | 20.000 | 0.667 |
| Microcrystalline cellulose | 13.200 | 0.440 |
| Safflower | 8.000 | 0.267 |

### Example 4. 1 g tablets - Daily dose 1 tablet

| **Name of component** | **mg / tablet** | **%** |
|---|---|---|
| N-acetyl-L-cysteine | 500.000 | 50.0000 |
| Hydroxypropyl methylcellulose | 250.000 | 25.0000 |
| Dibasic calcium phosphate | 120.000 | 12.0000 |
| Microcrystalline cellulose | 70.335 | 7.0335 |
| Promelase | 30.000 | 3.0000 |
| Magnesium stearate | 10.500 | 1.0500 |
| Levilite | 10.500 | 1.0500 |
| Shellac | 8.000 | 0.8000 |
| E110 colouring | 0.587 | 0.0587 |
| E 124 colouring | 0.078 | 0.0078 |

### Example 5. 400 mg capsules - Daily dose: 2 capsules

| **Name of component** | **mg / capsule** | **%** |
|---|---|---|
| N-acetyl-L-cysteine | 250.000 | 62.500 |
| Microcrystalline cellulose | 64.000 | 16.000 |
| Concentrated peptidase (Serrazimes®) | 50.000 | 12.500 |
| Polyvinylpyrrolidone | 28.000 | 7.000 |
| Magnesium stearate | 4.000 | 1.000 |
| Levilite | 4.000 | 1.000 |

### Example 6. 400 mg capsules - Daily dose: 2 capsules (Reference example)

| **Name of component** | **mg / capsule** | **%** |
|---|---|---|
| N-acetyl-L-cysteine | 250.000 | 62.500 |
| Microcrystalline cellulose | 64.000 | 16.000 |
| Serratiopeptidase | 50.000 | 12.500 |
| Polyvinylpyrrolidone | 28.000 | 7.000 |
| Magnesium stearate | 4.000 | 1.000 |
| Levilite | 4.000 | 1.000 |

## Claims

1. Use of N-acetyl-L-cysteine combined with Promelase for the preparation of compositions for the treatment and prevention of symptoms associated with acute and chronic inflammations and infections of the upper and lower airways.

2. Use as claimed in claim 1, wherein the symptoms associated with acute and chronic inflammations and infections of the upper and lower airways include altered trophism and oxidation of the mucous membranes; hypersecretion, abnormal viscoelasticity and reduced transport of bronchial and nasal mucus; coughing; local inflammation, oedema and pain; and antibiotic-resistant infections.

3. A combination of N-acetyl-L-cysteine and Promelase for use in the treatment and prevention of symptoms associated with acute and chronic inflammations and infections of the upper and lower airways.

## Patentansprüche

1. Verwendung von N-Acetyl-L-cystein in Kombination mit Promelase zur Herstellung von Zusammensetzungen für die Behandlung und Vorbeugung von Symptomen, die mit akuten und chronischen Entzündungen und Infektionen der oben und unteren Atemwege zusammenhängen.

2. Verwendung wie in Anspruch 1 beansprucht, wobei die mit akuten und chronischen Entzündungen und Infektionen der oberen und unteren Luftwege verbundenen Symptome veränderte Trophik und Oxidation der Schleimhäute; Hypersekretion, abnormale Viskoelastizität und verminderten Transport von bronchialem und nasalem Schleim; Husten; lokale Entzündung, Ödem und Schmerzen; und Antibiotika-resistente Infektionen einschießen.

3. Kombination von N-Acetyl-L-cystein und Promelase zur Verwendung bei der Behandlung und Vorbeugung von Symptomen, die mit akuten und chronischen Entzündungen und Infektionen der oberen und unteren Atemwege zusammenhängen.

## Revendications

1. Utilisation de la N-acétyl-L-cystéine combinée avec de la promélase pour la préparation de compositions destinées au traitement et à la prévention des symptômes associés à des inflammations et des infections aiguës et chroniques des voies aériennes supérieures et inférieures.

2. Utilisation selon la revendication 1, dans laquelle les symptômes associés à des inflammations et des infections aiguës et chroniques des voies aériennes supérieures et inférieures comprennent un trophisme et une oxydation modifiés des muqueuses ; une hypersécrétion, une viscoélasticité anormale et un transport réduit du mucus bronchique et nasal ; une toux ; une inflammation locale, un oedème et une douleur ; et les infections résistantes aux antibiotiques.

3. Combinaison de N-acétyl-L-cystéine et de promélase destinée à une utilisation dans le traitement et la prévention des symptômes associés à des inflammations et des infections aiguës et chroniques des voies aériennes supérieures et inférieures.
